# EUROPEAN PATENT APPLICATION

(11) **EP 2 514 426 A1**
(43) Date of publication of application: **24.10.2012**
(21) Application number: 10837083.4
(22) Date of filing: 14.12.2010
(51) Int. Cl.: A61K 31/7034

(54) **COMPOUNDS HAVING ANTI-INFLAMMATORY ACTIVITY**

(30) Priority: 15.12.2009 ES 200931169
(71) Applicant: Consejo Superior De Investigaciones Científicas (CSIC), 28006 Madrid (ES)
(72) Inventor: ESPIN DE GEA, Juan Carlos, E-30100 Murcia (ES); MORALES SÁNCHEZ, Juan Carlos, E-41092 Sevilla (ES); MEDINA MENDEZ, Isabel, E-36208 Vigo (Pontevedra) (ES)
(74) Representative: Fleck, Barbara
(86) International application number: PCT/ES2010/070826
(87) International publication number: WO 2011/073482

(57) **Abstract**

Compounds of general formula (I) for the treatment of inflammatory processes involved in numerous diseases such as intestinal inflammatory diseases. The invention moreover refers to compounds included within such general formula and to the procedure of the obtainment thereof.

## Description

This invention relates to the use of compounds of general formula (I) for the treatment of inflammatory processes involved in many diseases such as intestinal inflammatory diseases. The invention also relates to a number of specific compounds included in this general formula, the process for obtaining them and their antioxidant activity in foods.

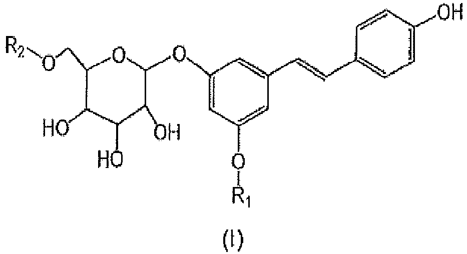

### STATE OF THE ART

Inflammation is a tissue process comprising a number of molecular, cellular and vascular phenomena having a defensive purpose against physical, chemical or biological aggression. The basic aspects apparent in the inflammatory process are first focalization of the response, which tends to circumscribe the area in which the aggressive agent is being fought. Secondly the inflammatory response is immediate, urgent and therefore predominantly non-specific, although it can encourage the subsequent development of a specific response. Thirdly, the inflammatory focus attracts immune cells from adjacent tissues. Thus inflammation arises with the defensive purpose of isolating and destroying the harmful agent, as well as repairing the damaged tissue or organ. However, the main problem arising from inflammation is that the defense is directed against both harmful and non-harmful agents in such a way that lesions can be caused in healthy tissues or organs.

Conventionally inflammation is regarded as comprising five cardinal signs: heat, rubor, tumor, pain and loss or diminution of function (functio laesa).

The type of treatment which should be applied to an inflammation depends on the nature of the affected area and the causes which have brought it about.

Inflammation has a fundamental part to play in the pathogenesis of many chronic diseases such as rheumatoid arthritis, asthma, type 2 diabetes, psoriasis, multiple sclerosis, atherosclerosis, intestinal inflammatory disease, chronic obstructive pulmonary disease, neurodegenerative diseases and cancer.

For example, inflammation is involved in both the genesis and development of atherosclerosis and the breakdown of atherosclerotic plaques, as well as in their stability and repair. Thus atherosclerosis must be regarded as an inflammatory disease for all purposes strictly related to its development with the thrombosis. Evidence shows that circulating levels of inflammatory/anti-inflammatory markers can predict an unfavorable cardiovascular response in both healthy subjects and patients with established heart disease. The factors leading to inflammation in atherogenesis are the so-called "conventional" risk factors such as smoking, hypercholesterolemia, arterial hypertension, obesity and diabetes. These factors have the endothelium as their target organ, hence the cytokines, which are capable of leaving the bloodstream, act to stimulate this endothelium and also leukocytes with the induction of many molecules adhering to the surface of the cells. Inflammation also contributes to the final stage causing fissure or erosion of atherosclerotic plaques. With regard to plaque stability/instability, nuclear transcription factor NF-kB has a fundamental part to play in the synthesis of products involved in plaque instability. Finally macrophages and the numerous enzyme family of the metaloproteinases induce break-up of the fibrous matrix and appearance of acute coronary syndrome through activating coagulation. This thrombotic process which appears as a response to the break-up of a plaque is associated with the formation of a thrombus as a result of which acute coronary syndrome occurs through consequent occlusion of the lumen of the vessel.

Another important involvement of inflammatory processes relates to the association between neuroinflammation and Alzheimer's disease, which has been proven through neuropathological and epidemiological investigations. Clinical/pathological and neuroimaging studies show that microglial inflammation and activation precede neuron damage, and that oxidative stress occurs prior to the cytopathology of Alzheimer's disease. Cerebral IL-1β, TGF-β and COX-2 are high in Alzheimer's disease. The epidemiological evidence and various experimental models show that pro-inflammatory conditions promote the development of Alzheimer's disease, while chronic anti-inflammatory treatment alters the incidence of this disease. Thus one current hypothesis is that the damage caused by the build-up of β-amyloid peptide is perhaps less than that caused by the inflammatory response prior to its build-up.

The relationship between inflammation and cancer is obvious. It has been stated that 20% of cancers develop as a chronic pro-inflammatory state. Cell mediators and effectors in inflammation are important constituents of the micro-environment of tumors. In some types of cancer inflammatory conditions precede the malignant transformation of cells. On the other hand, in other types of cancer there occur oncogenic changes which induce an inflammatory micro-environment which promotes tumor growth. At the present time the relationship between inflammation and cancer is firmly established and the identification of new target molecules relating to inflammation could improve the diagnosis and treatment of some types of cancer.

When inflammation affects the gastrointestinal tract we talk about inflammatory intestinal diseases, a term which generally applies to two independent clinical conditions, namely ulcerative colitis and Crohn's disease. There is also indeterminate colitis, although this is not regarded as an independent condition.

Ulcerative colitis is a chronic inflammatory disease which affects the mucosa of the colon in a diffuse and continuous way to various extents. Most patients with ulcerative colitis are treated medically instead of surgically.

Crohn's disease is also a chronic inflammatory disease, but unlike ulcerative colitis it may affect any part of the digestive tract, from the mouth to the anus. Even when lesions may begin at the surface, the inflammatory process extends through the intestinal wall to the drainage lymphatic nodes. Most patients with Crohn's disease undergo surgery at some time, but continuing medical treatment is normal.

Indeterminate colitis refers to the chronic intestinal disease which only affects the colon, while ruling out infectious colitis and other causes of colitis. Its clinical, anatomical/pathological and endoscopic characteristics do not allow it to be classified with ulcerative colitis, or Crohn's disease.

Of the most frequent functional digestive disturbances mention should be made of irritable bowel syndrome (IBS), which has a major socioeconomic impact. At the present time IBS is considered to be a precursor of an intestinal inflammatory disease such as ulcerative colitis. An inflammatory condition in the intestinal mucosa of patients with IBS, with high production of the pro-inflammatory cytokines TNFα and IL-6 and a fall in the anti-inflammatory IL-10 has been described. Myeloperoxidase is also increased in leukocytes of patients with IBS in comparison with healthy individuals. IBS affects all ages, more women than men, and all human races. The prevalence of IBS in Spain amounts to around 10% of the population when more than two criteria of those described by Manning are considered, and 3.3% on the basis of the Rome II criteria. There are no data on the prevalence of IBS according to the new Rome III criteria.

Chronic intestinal inflammatory diseases (IID) show periods of activity (outbreaks) of variable intensity and severity, with periods of inactivity (remission), for which treatment is essentially determined by the severity of clinical manifestations and their anatomical extent. The main aim is to maintain equilibrium between greater efficacy and fewer secondary effects. The present pharmacological treatment of IID comprises the use of antiinflammatories (aminosalicylates and corticoids) and immunomodulators.

In the treatment of acute attacks of ulcerative colitis glucocorticosteroids such as prednisone acetate and prednisolone acetate are almost invariably used. Once remission has been achieved, sulfasalazine is the maintenance treatment of choice for the treatment of ulcerative colitis. However, this drug has many secondary effects due mainly to absorption of the sulfapyridine residue from the colon. Recently compounds containing only 5-aminosalicylic acid have been developed; these compounds are as effective as sulfasalazine and do not have the secondary effects of sulfapyridine, but they have their own secondary effects, especially diarrhea.

In active severe Crohn's disease glucocorticosteroids are the treatment of choice, but ideally only to achieve remission, after which treatment should cease. However, all too frequently there is no satisfactory remission of the disease and glucocorticosteroids may be required to maintain control of symptoms. Sulfasalazine is also useful in less severe cases, in particular for the disease affecting the colon. Very frequently in Crohn's disease however primary medical treatment of the process of the disease is ineffective, symptomatic treatment, that is to say analgesics for pain and opiates for diarrhea, are of value. Immunomodulators such as azathioprin are also commonly used in Crohn's disease with a view to reducing the proliferation of B and T lymphocytes and the primary immune response, as well as the function of the lymphocytes and "natural killer" cells. One increasingly common treatment for Crohn's disease and ulcerative colitis, despite its extremely high costs, comprises the administration of monoclonal antibodies especially against alpha type tumor necrosis factor (TNFα). These are therapeutic agents having a selective anti-inflammatory action. The basic effect is brought about by blocking TNFα and reducing other pro-inflammatory cytokines such as IL-6, and the migration of leukocytes into the intestine.

Acute indeterminate colitis (IC) is handled in the same way as severe ulcerative colitis, although clinical developments may finally cause the patient to be classified as one with Crohn's disease. At the present time the diagnosis of IC is provisional and is only considered when the inflammatory disease is localized in the colon and no conclusive distinction can be made with regard to ulcerative colitis and Crohn's disease.

The present treatment for colitis and Crohn's disease is unfortunately associated with severe and frequent secondary effects.

50% of patients treated with aminosalicylates (doses used from 500 mg to 1 g) present with dose-dependent secondary effects (headaches, nausea, abdominal pain, hematological changes, liver and/or kidney intoxication, etc.).

Corticoids (prednisone, prednisolone, budesonide) are the drugs of choice for outbreaks of IID. These corticoids have high bioavailability (50-80%), which encourages the appearance of secondary effects. At the present time strategies are being sought to increase their luminal effects in the intestine and reduce their toxicity. Secondary effects may derive from the sudden removal of corticoids and from their prolonged use. The most serious acute complication deriving from the sudden withdrawal of steroids is acute adrenal failure. Other acute secondary effects are arterial hypertension, hypercholesterolemia, fluid retention, weight gain, glucose intolerance, leukocytosis, insomnia, emotional lability, psychotic conditions, osteoporosis, glaucoma, slow growth in children, etc. Although the majority of effects should revert when corticoids are withdrawn, unfortunately they are often frequent, severe and lasting.

Immunomodulators (azathioprin, mercaptopurine) give rise to adverse effects which make it necessary to withdraw treatment in 15-30% of patients. The most common adverse effects are nausea and vomiting, pancreatitis, fever, hepatotoxicity and leukopenia. Other long-term effects are an increase in viral infections, *Herpes zoster,* hepatitis A/B, pneumonias, etc. Another immunomodulator used is methotrexate, which inhibits lymphocyte proliferation and may modulate the cytokine profile. The main secondary effect is hepatotoxicity, mucositis, medullary aplasia, osteopathy, opportunistic infections, etc.

Cyclosporine is another immunomodulator which acts by inhibiting the pro-inflammatory cytokines IL-2 and IFN-γ, TNFα and IL-4. The most important secondary effect is nephrotoxicity, nausea, tremor, headaches and gingival hyperplasia.

Infliximab is an anti-TNFα monoclonal antibody with which there is more clinical experience. Secondary effects include allergic reactions during infusion (intravenous over several hours), headache, nausea, urticaria, chest pain and infectious complications. Despite its effectiveness especially in fistulizing Crohn's disease, complications have nevertheless been described, worsening conditions of stenosis in the terminal and pre-terminal ileum.

Functional foods (FF) are those foods which have been developed not only for their nutritional characteristics but also to fulfill a specific function such as to improve health and reduce the risk of contracting diseases. The attainments most mentioned in the scientific literature and in the marketing of food products are improved gastrointestinal functions, the provision of redox and antioxidant systems, as well as the modification of macronutrient metabolism.

Functional foods are prepared by increasing active components of actual benefit to health through various techniques. One of the compounds of most interest at the present time is resveratrol.

Resveratrol is a phenol compound. The chemical structure of the phenol compounds comprises at least one aromatic ring and one hydroxyl group. Among the phenol compounds resveratrol is a stilbene, characterized in that this group of phenol compounds have a structure in which 2 phenol rings are joined through two carbon atoms (C₆-C₂-C₆). Resveratrol is present in grapes and derived products such as wine, and in other foods, although in much smaller quantities, such as peanuts and some berries. In these foods it is found in the free state or as a piceid (resveratrol-3-O-glucoside). This compound has antioxidant, anti-inflammatory and anti-tumoral properties which prolong cell longevity. Therefore foods and drinks which contain this substance are regarded as being healthy or recommendable for health.

Patent Application WO2007020673 describes the use of some compounds for the treatment of ophthalmological diseases, and these compounds include trans-resveratrol-3,5-*O*-diglucoside.

Resveratrol has chemopreventive activity against cancer in tests representing three main stages of carcinogenesis. That is the authors have discovered that the compound:
1. acts as an antioxidant and antimutagen and induces enzymes which metabolize drugs;
2. mediates anti-inflammatory effects and inhibits cyclooxygenase (COX) and hydroperoxidase; and
3. induces cell differentiation in human promyelocytic leukemia. In addition to this, as indicated above, resveratrol has been studied extensively on account of its correlation with the cardiovascular usefulness of red wine.

Recently it has been demonstrated that resveratrol inhibits the transcription of COX-2 (see ES2266271), and also inhibits the enzyme activity of COX-1. Known chemopreventive agents against cancer include non-steroid anti-inflammatory drugs (NSAID) such as indomethacin, aspirin, pyroxicam and sulindac, all of which inhibits cyclooxygenase. The inhibiting activity of COX is important in the chemoprevention of cancer because COX catalyses the conversion of arachidonic acid into pro-inflammatory substances such as prostaglandins which can stimulate the growth of tumor cells and suppress immunological vigilance. In addition to this COX can activate carcinogens in forms which damage genetic material. Some researchers have suggested new chemopreventive agents against cancer through the evaluation of plant extracts to find a potential COX inhibiter. Among these there is an extract derived from *Cassia quinquangulata* Rich (*Leguminosae*), a powerful COX inhibiter, and trans-resveratrol has been identified as the active compound (see Mannila et al., Phytochemistry 33:813, 1998). Neurological uses have also been proposed for resveratrol (see Lee et al., Society for Neuroscience Abstracts 20(1-2): 1648, 1994).

### DESCRIPTION OF THE INVENTION

This invention provides compounds which can be used to prepare a pharmaceutical or food composition for the treatment or prevention of inflammatory processes.

Thus a first aspect of this invention relates to the use of a compound of general formula (I) for the preparation of a pharmaceutical or food composition (including those described as functional foods) for the treatment and/or prevention of inflammatory processes: 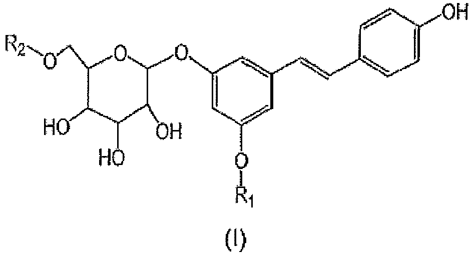 in which:
R₁ is hydrogen or the group having the general formula (II): 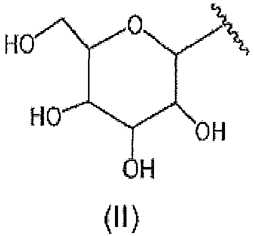
R₂ is hydrogen or forms an acyl group together with oxygen (-OCO-R₃), R₃ is a (C₁-C₂₂) alkyl or (C₂-C₂₂) alkenyl group, and
when R₂ is hydrogen R₁ represents the group of general formula (II).

In this invention the term "alkyl" relates to straight or branched aliphatic chains having 1 to 22 carbon atoms, for example methyl, ethyl, *n*-propyl, *i*-propyl, *n*-butyl, t-butyl, *sec*-butyl, n-pentyl, n-hexyl, etc. Preferably the alkyl group has between 2 and 10 carbon atoms, and more preferably it has between 3 and 7 carbon atoms.

In this invention the term "alkenyl" refers to straight or branched unsaturated aliphatic chains which have 2 to 22 carbon atoms, and which have between one and six unsaturations, for example vinyl, allyl, oleyl, linoleyl, etc.

When R₃ is an alkyl group R₂ forms an ester group together with the oxygen to which it binds and more preferably an ester group of a saturated fatty acid of the type (HO-CO-(CH)ₙ-CH₃), where n+1 is the number of carbon atoms in the aliphatic chain described above.

When R₃ is an alkenyl group, R₂ will form an ester group together with the oxygen to which it binds and more preferably an ester group of an unsaturated fatty acid. Depending upon the number of unsaturations these may be monounsaturated fatty acids, that is those with just one double bond, or polyunsaturated fatty acids with two or more unsaturations. These unsaturated fatty acids may also have a *cis* or *trans* configuration.

Another preferred embodiment of this invention comprises the use of a compound of general formula (I) in which R₂ is hydrogen and R₁ represents the compound of general formula (II).

In another preferred embodiment of this invention the pyranose rings forming part of structures (I) and (II) represent sugars or monosaccharides which may be selected independently from one another from glucose, galactose, mannose or allose, but preferably the rings are glucose. Furthermore, the unions between these monosaccharides included in general formulas (I) and (II) with the corresponding phenol ring may be alpha or beta, but preferably the unions will be beta.

In another preferred embodiment of this invention the compounds of formula (I) used are selected from the list comprising *trans*-resveratrol-3,5-di-*O*-β-D-glucopyranoside, *trans*-resveratrol-3-*O*-(6'-*O*-butanoyl)-β-D-glucopyranoside (also known as trans-piceid-butyrate or BUT) and *trans*-resveratrol-3-*O*-(6'-*O-*octanoyl)-β-D-glucopyranoside (also known as *trans-*piceid-octanoate or OCT).

In another preferred embodiment of this invention, the inflammatory diseases are intestinal.

By "inflammatory diseases" are meant diseases which progress through acute and/or chronic inflammatory processes of high or mild intensity related to cancer, autoimmune, cardiovascular and neurodegenerative diseases, intoxications, infections (by microorganisms such as fungi, bacteria, etc., or viruses), as well as others known to those skilled in the art. For example, bacterial infectious processes may include those caused by *E*. *coli O157*, *Salmonella enteritidis* or *Listeria monocytogenes.* In particular by "intestinal inflammatory diseases" reference is made in this invention to those which occur in the digestive tract, especially the small and large intestines, which may give rise to many symptoms in individuals suffering from it, such as for example weight loss, blood in feces and/or diarrhea among others, which cause a considerable deterioration in their quality of life. These diseases may be selected from the list comprising irritable bowel syndrome, indeterminate colitis, ulcerative colitis and Crohn's disease.

As demonstrated in this invention, through the use of compounds of general formula (I) particular effects and markers associated with intestinal inflammation, such as weight loss, hidden blood in feces, myeloperoxidase activity, shortening of the colon, prostaglandins, tumor necrosis factor receptor (TNFR1), macrophage inflammation proteins (MIP), T-cell attracting cytokines (MIG), interleukin-6 (IL-6) or COX-2 can be reduced. Furthermore, through use of the compounds of general formula (I) a reduction in systemic inflammation represented by a reduction in the acute phase inflammation markers haptoglobin and fibrogen has also been described, and this also indicates its action on inflammatory processes other than those in the digestive tract.

In this invention by "food composition" is meant a foodstuff or food supplement included among those known as "nutraceuticals" or "functional foods" which have a beneficial effect on health. Likewise this term can be applied to extracts or chemical compounds obtained from common foods. Functional foods are normally used in nutritional mixtures and in the pharmaceutical industry. In the same way that some foods can be classified as functional foods, some nutritional supplements are also classified in this way, such as for example fatty acids such as the omega-3 derivatives of fish oil and some plants or antioxidants and vitamins.

In this invention the compounds described above can be used in foods (including those labeled as being functional) or nutraceuticals, not only to prevent the appearance of inflammatory processes but also to improve the body's defenses as part of the immune system (IL-3).

A second aspect of this invention relates to a compound of general formula (III), which comprises the compound of general formula (I) when R₁ is hydrogen and R₂ is an acyl group (-OCO-R₃): 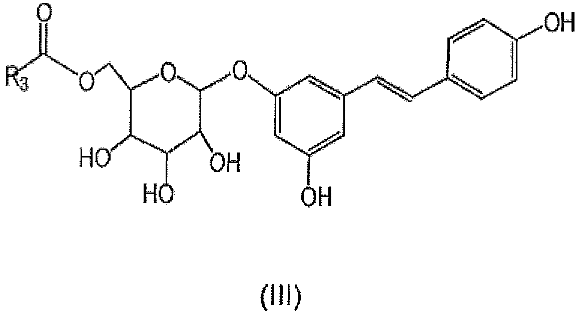 in which: R₃ is as described previously.

In a preferred embodiment of the compounds of general formula (III), R₃ is a (C₂-C₁₀) alkyl group, and more preferably R₃ is a (C₃-C₇) alkyl group.

In another preferred embodiment of this invention the pyranose ring forming part of structure (III) represents a sugar or monosaccharide which may be selected from glucose, galactose, mannose or allose, more preferably the ring is glucose. Furthermore, the union between this monosaccharide present in general formula (III) and the phenol ring may be alpha or beta, more preferably the union is beta.

In another preferred embodiment the compounds of general formula (III) according to the invention are trans-resveratrol-3-*O*-(6'-*O*-butanoyl)-β-D-glucopyranoside or resveratrol-3-*O*-(6'-*O*-octanoyl)-β-D-glucopyranoside.

A third aspect of this invention relates to the process for obtaining compounds of general formula (III) according to the invention, which comprises:
a. mixing a compound of formula (IV) with a compound of formula (V): R₃-COO- R₄ in the presence of a lipase,
   in which: R₃ is as described above and R₄ is hydrogen or a group activating formation of the ester bond.

The process according to the invention may be seen in the following diagram:

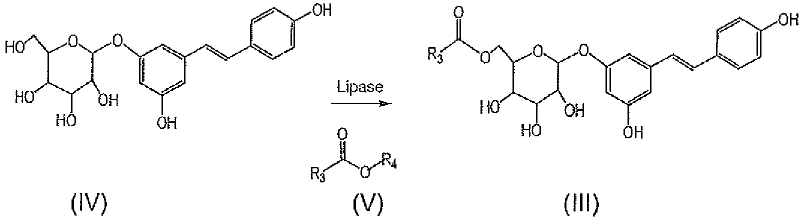

In a preferred embodiment compound (IV) is a glucoside, mannoside, alloside or galactoside derivative, more preferably the compound is trans-resveratrol-3-*O*-β-D-glucopyranoside.

The lipase used as a biocatalyst in the process described may be the lipase from *Candida antarctica, Aspergillus niger, Candida rugosa, Pseudomonas cepacia, Rhizomucor miehei,* and preferably the lipase from *Thermomyces lanuginosus.* Even more preferably this lipase will be immobilized, preferably in silica.

In a preferred embodiment of the process according to the invention mixing takes place in an organic solvent such as for example, but without being restricted to, acetone, diethylether, diisopropylether, methyl t-butylether, t-butanol, t-amyl alcohol or t-pentyl alcohol or mixtures of one of these with hexane, pentane, cyclohexane, pyridine or toluene. Preferably the solvent will be t-butanol, and the reaction mixture can be heated to a temperature of between 30 and 70°C, preferably between 55 and 65°C, and preferably to 60°C. In a preferred embodiment of the process according to the invention, the R₄ group activating formation of the ester bond is the ethyl group or preferably the vinyl group, i.e. formula (V) will be as follows, in which R₄ contains a vinyl group:

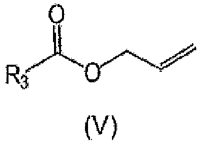

Another aspect of this invention relates to the use of compounds of general formula (III) for the preparation of a medication.

A fourth aspect of this invention relates to a pharmaceutical composition comprising at least one compound of general formula (I) and a pharmaceutically acceptable vehicle. Optionally this composition may comprise another active substance.

"Pharmaceutically acceptable vehicles" which may be used in these compositions are vehicles known to those skilled in the art.

As examples of pharmaceutical preparations which include any solid composition (tablets, pills, capsules, granules, etc.) or liquid compositions (solutions, suspensions, gels, emulsions, etc.) for oral, topical or parenteral administration, administration will preferably be oral.

In another aspect this invention relates to a method for the treatment and prevention of inflammation in mammals, preferably humans, comprising the administration of a therapeutically effective quantity of a composition of general formula (I) as described above. Preferably administration of the composition will take place orally.

In the meaning used in this description the term "therapeutically effective quantity" refers to the quantity of composition (I) calculated to produce the desired effect and in general will be determined among other things by the specific characteristics of the composition, and patient's age, condition and previous history, the severity of the change or condition, and the route and frequency of administration.

Preferably, in this method of treatment the condition will be selected from conditions or diseases related to the intestinal tract and/or systemic inflammations, and more preferably from intestinal inflammations.

Another aspect of this invention relates to the use of the compound of general formula (III) as a system for delivering and achieving a higher concentration of resveratrol in the large intestine ("drug delivery system"). A major problem with resveratrol is its fast absorption in the anterior portions of the intestinal tract and its extensive conjugation by phase II enzymes (glucuronyl-transferases, sulfotransferases, etc.) to produce their corresponding metabolites resveratrol-glucuronides and resveratrol-sulfates, among others, which circulate in the blood. Resveratrol undergoes extensive enterohepatic metabolism which prevents it from reaching distal parts of the intestine, including the ileum and colon, and therefore renders its action difficult in pathological processes (including inflammatory processes) taking place in these areas.

Another aspect of this invention relates to the use of the compound of general formula (III) to modulate intestinal microbiota, increasing the population of bifidobacteria and lactobacilli and decreasing the increase in opportunistic pathogens such as for example enterobacteria.

Another aspect of this invention relates to the use of the compound of general formula (III) for the preparation of a foodstuff composition. Preferably the foodstuff composition may be selected from a foodstuff, food supplement, functional foodstuff or nutraceutical.

Another aspect of this invention relates to a foodstuff composition comprising at least one compound of general formula (I), more preferably at least one compound of general formula (III).

Another aspect of this invention relates to use of the compound of general formula (III) as an antioxidant. More preferably as an antioxidant in foods.

Throughout the description and the claims the word "comprises" and its variants will not rule out other technical characteristics, additives, components or steps. To those skilled in the art other objects, advantages and characteristics of the invention will be apparent partly from the description and partly from the practice of the invention. The following examples and drawings are provided by way of illustration, and they are not intended to restrict this invention.

### DESCRIPTION OF THE FIGURES

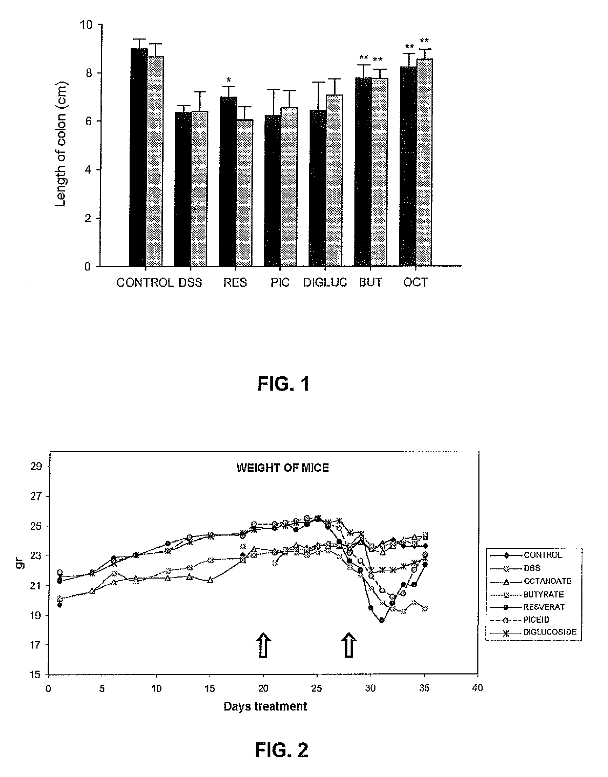

**FIGURE 1****.** Effect of administering resveratrol and its derivatives along the length of the colon through treatment with 1% DSS. Significant difference from the group with DSS (*P<0.05; **P<0.01). The length of the colon evaluated after 8 days administration of DSS (dark bar) and 6 days after interruption of the treatment with DSS (recovery, light bar). Dose equivalent to 10 mg for a 70 kg individual (Human Equivalent Dose, HED). C, control group; DSS, control group with inflammation induced by DSS; OCT, *trans-*piceid-octanoate; BUT, *trans*-piceid-butyrate; RES, *trans*-resveratrol; PIC, *trans*-piceid; DIGLUC, *trans-*resveratrol-3,5-*O*-diglucoside. The results are the mean of two independent tests with eight mice per group in each one of these tests. DSS is sodium dextran sulfate.
**FIGURE 2****.** Change in weight in animals in response to treatment with DSS and the effect caused by different derivatives of resveratrol (HED = 10 mg). The arrows indicate the start of administration of DSS (day 20) and the end of administration (day 28). The effect of the derivatives on the recovery of the animals after the suspension of treatment with DSS was monitored from day 29 to 35. The results are the mean of two independent tests with eight mice per group in each of these tests.

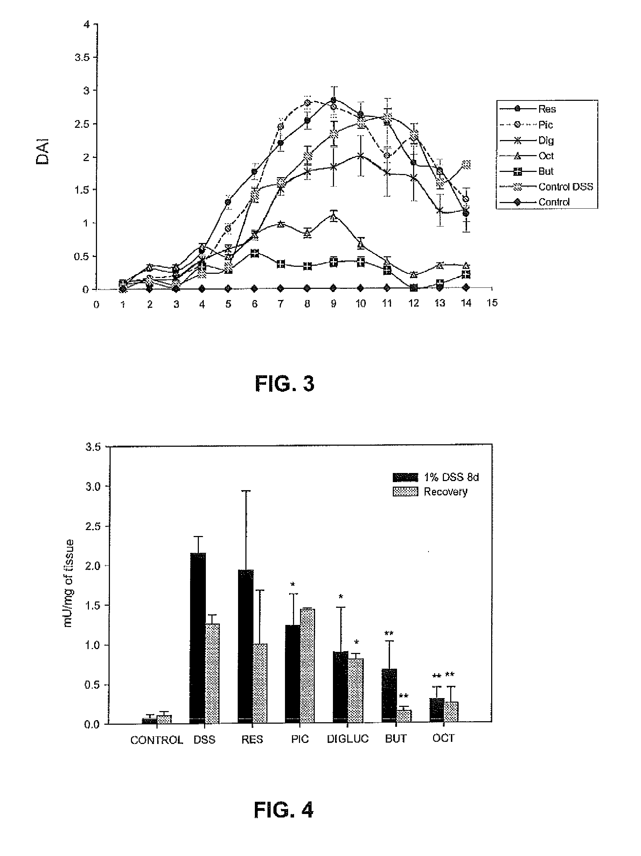

**FIGURE 3****.** Effect of resveratrol derivatives on the change in DAI following the administration of DSS. The changes over 14 days (8 days with DSS, and 6 subsequent days of recovery) are shown. The results are an average of two independent tests with eight mice per group in each of these tests.
**FIGURE 4****.** Myeloperoxidase activity in colonic mucosa after the administration of DSS in the presence and absence of resveratrol derivatives (HED = 10 mg). The asterisk indicates a significant difference in comparison with the group with DSS (P<0.05). Evaluated after 8 days with administration of DSS (dark bar) and 6 days after interruption of the treatment with DSS (recovery, light bar). The results are the mean of two independent tests with eight mice per group in each of these tests.

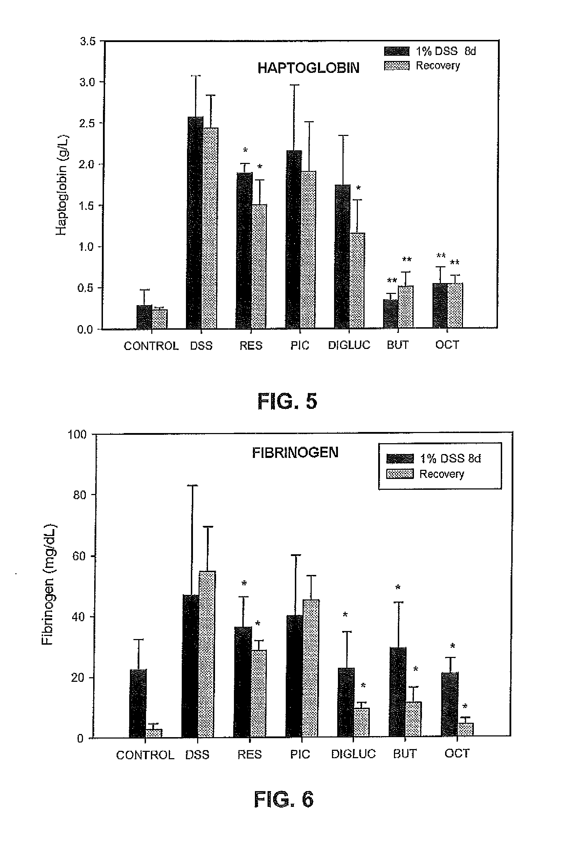

**FIGURE 5****.** Effect of resveratrol derivatives (HED = 10 mg) on blood haptoglobin levels. The asterisk indicates a significant difference in comparison with the group with DSS (P<0.05). Evaluated after 8 days administration of DSS (dark bar) and 6 days after interruption of the treatment with DSS (recovery, light bar). The results are the mean of two independent tests with eight mice per group in each of these tests.
**FIGURE 6****.** Effect of resveratrol derivatives (HED = 10 mg) on blood fibrinogen levels. The asterisk indicates a significant difference in comparison with a group with DSS (P<0.05). Evaluated after 8 days administration of DSS (dark bar) and 6 days after interruption of the treatment with DSS (recovery, light bar). The results are the mean of two independent tests with eight mice per group in each of these tests.

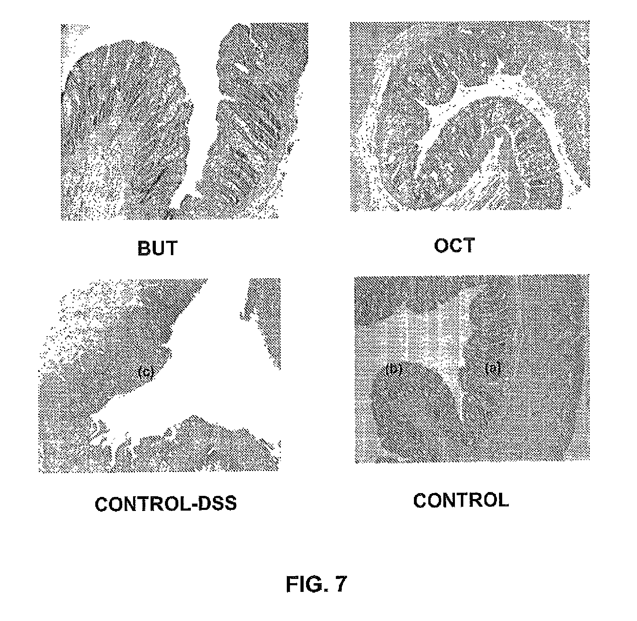

**FIGURE 7****.** Effect of the administration of resveratrol derivatives (HED = 10 mg) on the colonic architecture of mouse colon samples after the administration of DSS. Hematoxylin-eosin stain (x100). **(a)** crypts, **(b)** epithelium, (c) cellular infiltration. The figure shows a representative example of the effect observed in all the samples analyzed.

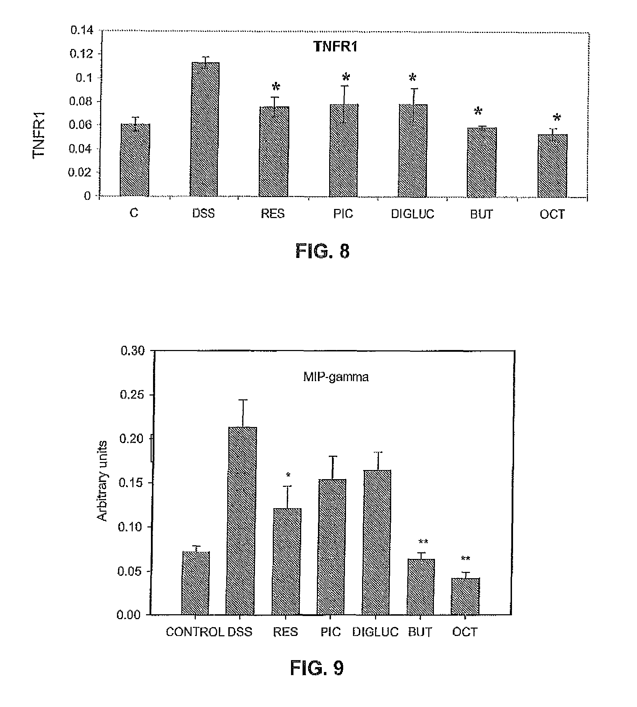

**FIGURE 8****.** Effect of administering resveratrol derivatives (HED = 10 mg) on the level of TNFR1 in colonic mucosa following the induction of inflammation with DSS. Determination after 8 days administration of DSS. The asterisk indicates a significant difference in comparison with the group with DSS (P<0.05). Values expressed in arbitrary units after measuring the intensity of the signals by densitometry. The results are the mean of two independent tests with eight mice per group in each of these tests.
**FIGURE 9****.** Effect of resveratrol derivatives (HED = 10 mg) on the level of gamma macrophage inflammatory protein (MIP-γ) in colonic mucosa after the induction of inflammation with DSS. The asterisk indicates a significant difference in comparison with the group with DSS (P<0.05). Values expressed in arbitrary units after measuring the intensity of the signals by densitometry. The results are the mean of two independent tests with eight mice per group in each of these tests.

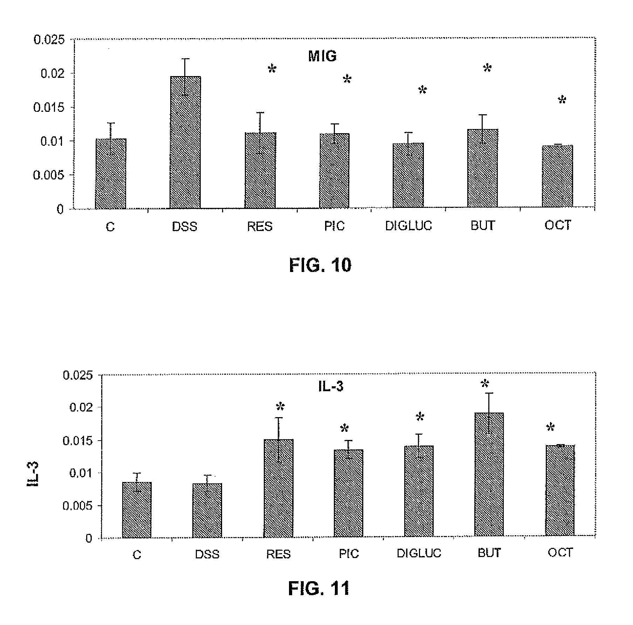

**FIGURE 10**. Effect of resveratrol derivatives (HED = 10 mg) on the level of protein of monocytes induced by gamma interferon (MIG) in colonic mucosa after the induction of inflammation with DSS. The asterisk indicates a significant difference in comparison with the group with DSS (P<0.05). Values expressed in arbitrary units after measuring the intensity of the signals by densitometry. The results are the mean of two independent tests with eight mice per group in each of these tests.
**FIGURE 11****.** Effect of resveratrol derivatives (HED = 10 mg) on the level of interleukin 3 (IL-3) in colonic mucosa after the induction of inflammation with DSS. The asterisk indicates a significant difference in comparison with the group with DSS (P<0.05). Values expressed in arbitrary units after measuring the intensity of the signals by densitometry. The results are the mean of two independent tests with eight mice per group in each of these tests.

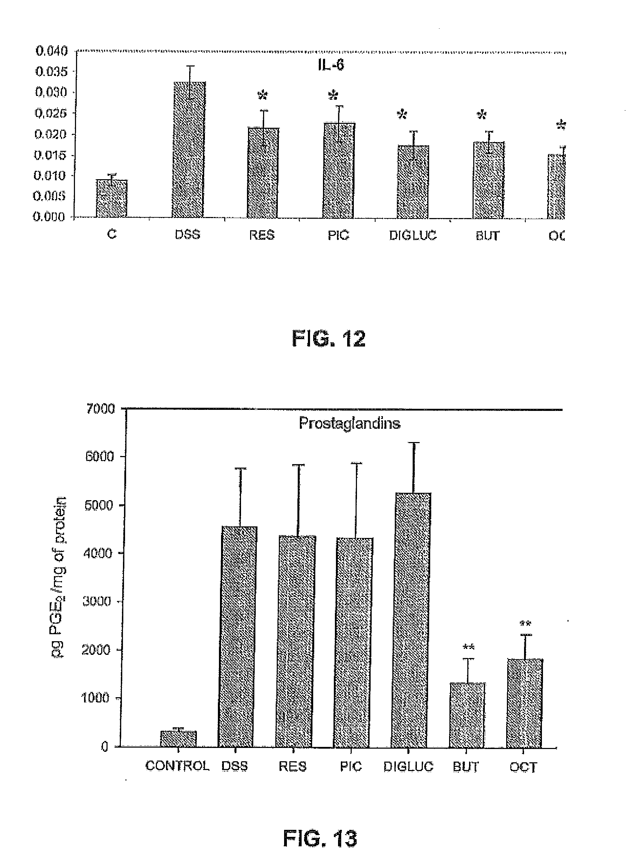

**FIGURE 12****.** Effect of resveratrol derivatives (HED = 10 mg) on the level of interleukin 6 (IL-6) in colonic mucosa after the induction of inflammation with DSS. The asterisk indicates a significant difference in comparison with the group with DSS (P<0.05). Values expressed in arbitrary units after measuring the intensity of the signals by densitometry. The results are the mean of two independent tests with eight mice per group in each of these tests.
**FIGURE 13****.** Effect of resveratrol derivatives (HED = 10 mg) on the level of prostaglandin E₂ (PGE₂) in colonic mucosa following induction of inflammation with DSS. The asterisk indicates a significant difference in comparison with the group with DSS (P<0.05). The results are the mean of two independent tests with eight mice per group in each of these tests.

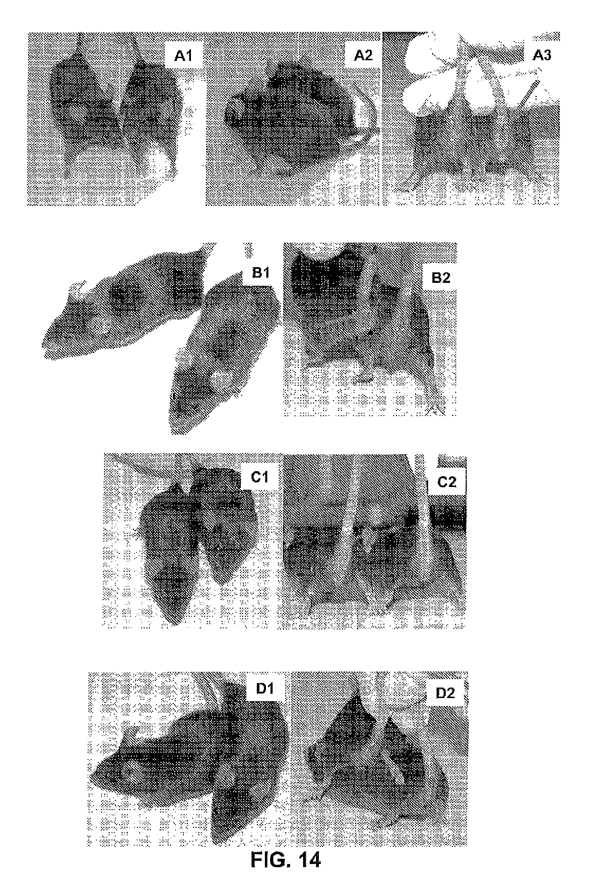

**FIGURE 14****.** Appearance of mice following the administration of 1% DSS for 8 days. The control-DSS (A) and mice which had previously consumed RES (B), BUT (C) and OCT (D) are shown as examples. The presence or absence of rectal hemorrhage (indicated by an arrow) is also shown in all cases. The mice previously fed with RES, BUT and OCT were of better appearance than the control-DSS, especially in the case of the mice fed with BUT and OCT, which showed no trace of rectal hemorrhage.

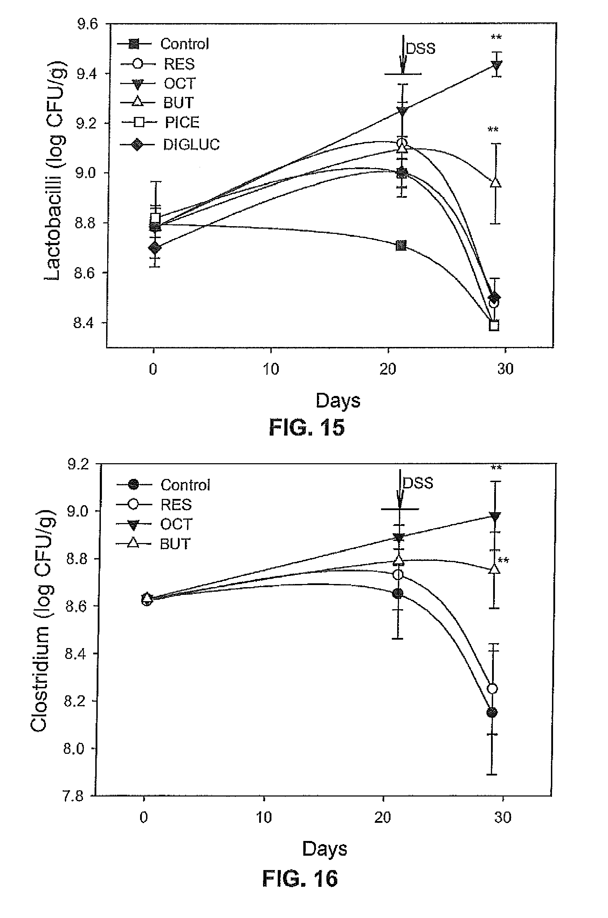

**FIGURE 15****.** Counts of bifidobacteria in feces before and after the administration of DSS, in mice receiving a diet supplemented with different compounds (HED = 10 mg). **Significant differences (P<0.01) in comparison with the control-DSS group.
**FIGURE 16****.** Counts of lactobacilli in feces before and after the administration of DSS in mice receiving a diet supplemented with different compounds (HED = 10 mg). **Significant differences (P<0.01) in comparison with the control-DSS group.

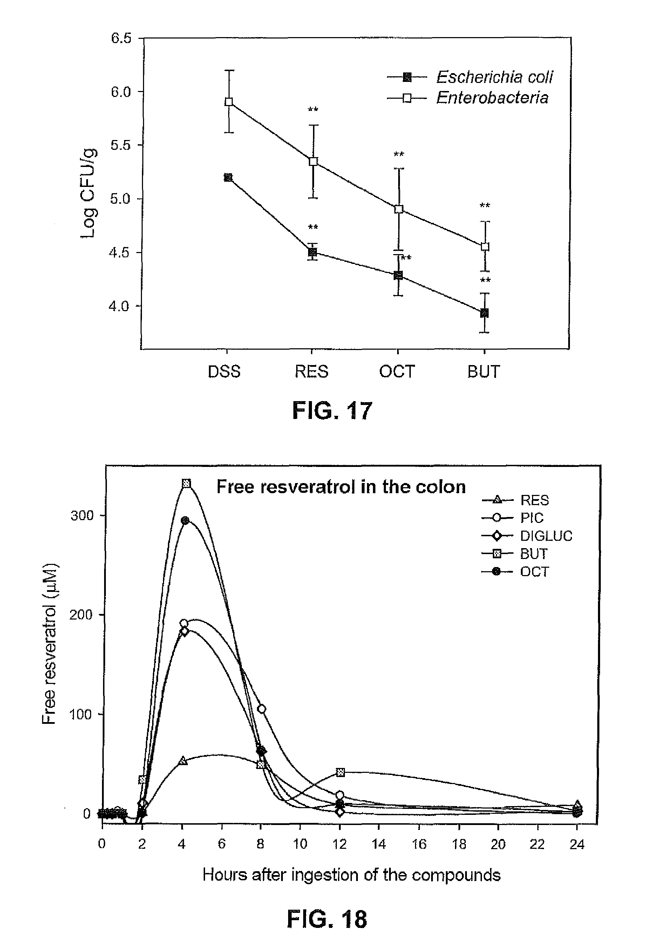

**FIGURE 17****.** Counts of clostridia in feces before and after the administration of DSS in mice receiving a diet supplemented with RES, BUT and OCT (HED = 10 mg). **Significant differences (P<0.01) in comparison with the control-DSS group.
**FIGURE 18****.** Counts of enterobacteria and *E*. *coli* in feces before and after the administration of DSS in mice receiving a diet supplemented with RES, BUT and OCT (HED = 10 mg). **Significant differences (P<0.01) in comparison with the control-DSS group.

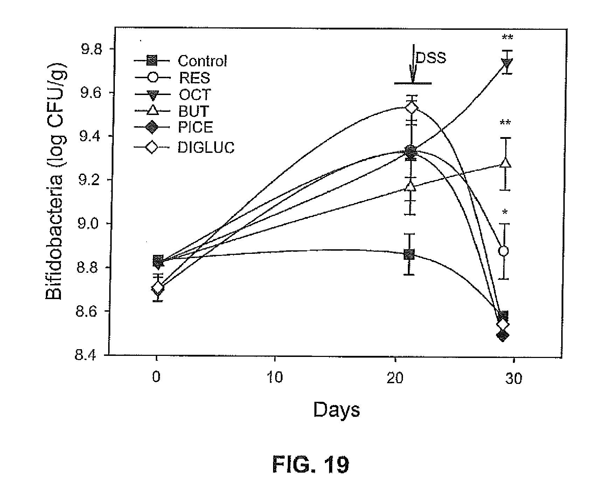

**FIGURE 19****.** Kinetics of the concentration of free resveratrol in the colon of healthy mice following the administration of resveratrol (RES), piceid (PICE), resveratrol-3,5-diglucoside (DIGLUC), butyrate-piceid (BUT) and octanoate-piceid (OCT).

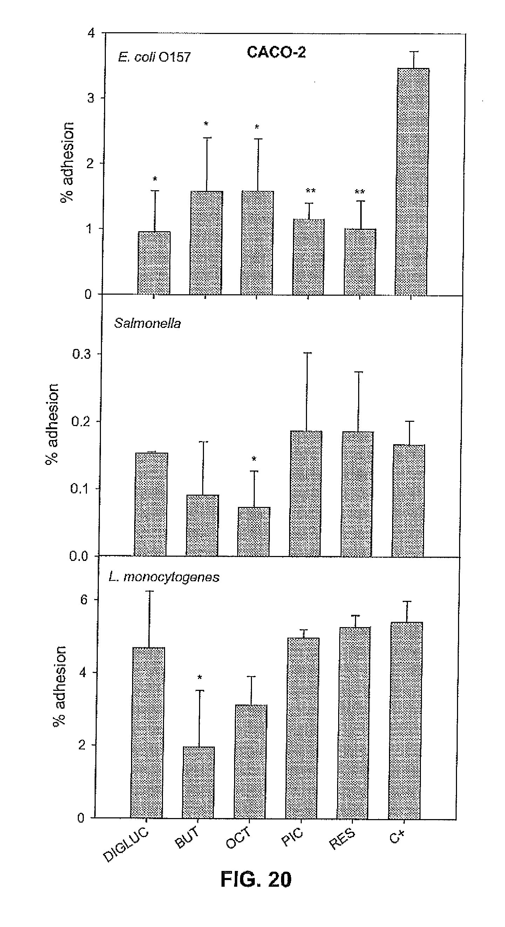

**FIGURE 20****.** Percentage adhesion of pathogenic bacteria to human colon Caco-2 cell cultures. Differences in comparison with the positive control C+ (bacteria only, no compounds). (*) P<0.05; (**) P<0.01.

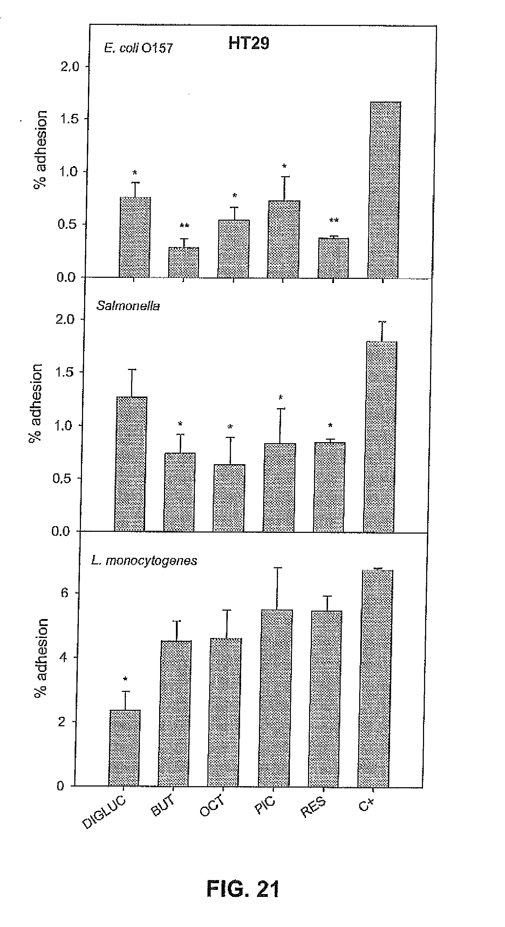

**FIGURE 21****.** Percentage adhesion of pathogenic bacteria to human colon HT-29 cell cultures. Differences in comparison with the positive control C+ (bacteria only, no compounds). (*) P<0.05; (**) P<0.01.

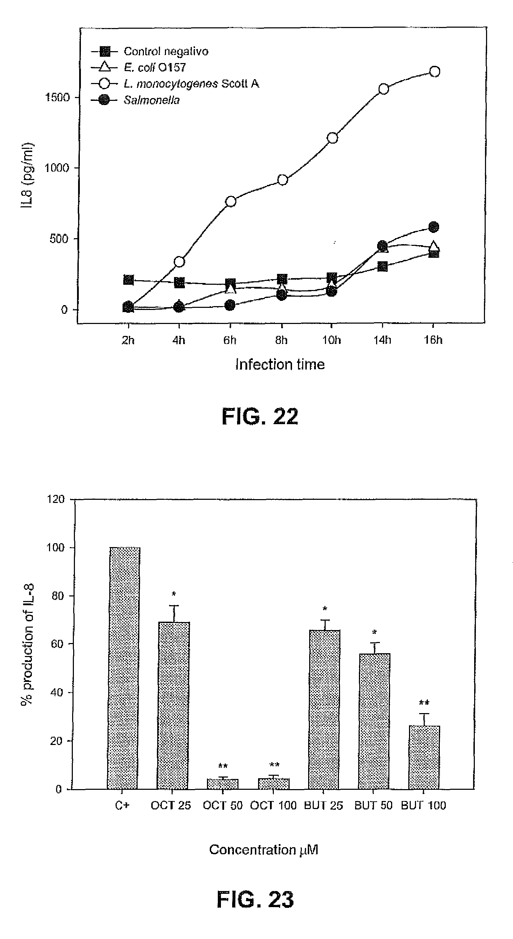

**FIGURE 22****.** Production of interleukin-8 in HT-29 cell culture as a response to inoculation with *Listeria monocytogenes* Scott A, *E*. *coli* O157 or *Salmonella.*
**FIGURE 23****.** Production of interleukin-8 in HT-29 cell culture as a response to inoculation of the pathogen *Listeria monocytogenes* Scott A. Differences with respect to the positive control C+ (Listeria only, no compounds). (*) P<0.05; (**) P<0.01.

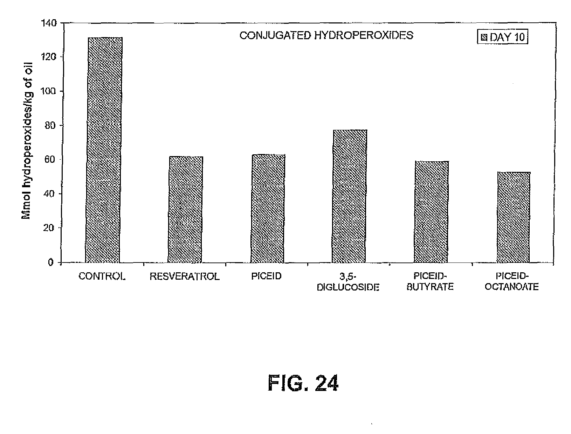

**FIGURE 24****.** Formation of conjugated hydroperoxides during the oxidation of emulsions of fish oil supplemented with 100 ppm of resveratrol, piceid, resveratrol-3,5-diglucoside, piceid-butyrate and piceid-octanoate. Values determined for the propagation period of the control (day 10 of oxidation).

### EXAMPLES

The invention is illustrated below through a number of tests carried out by the inventors which reveal the effectiveness of the compounds according to the invention.

### EXAMPLE 1.- SYNTHESIS OF COMPOUNDS OF GENERAL FORMULA (I)

In general these compounds are not of natural origin like resveratrol or piceid.

### Synthesis of the compound resveratrol-3,5-O-diglucoside (trans-resveratrol-3,5-di-O-β-D-glucopyranoside, DIGLUC).

The strategy used for the synthesis of resveratrol-3,5-O-diglucoside is similar to that used by Zhang et al. (see Zhaojun Zhang, Biao Yu, Richard R. Schmidt, Synthesis, 2006, No. 8, 1301-1306). t-Butyl-dimethylsilyl-4-*O*-resveratrol, a partly protected intermediate which is obtained by the reaction of resveratrol with t-butyl-dimethylsilyl chloride (TBSC1), was first prepared. This derivative was then caused to react with a glucosyl donor. In our case 2,3,4,6-tetra-*O*-benzoyl-D-glucopyranosyl trichloroacetamidate was used instead of the trifluoroacetamidate derivative used by Zhang et al. The trichloroacetamidate derivative is easier to prepare and cheaper, and provides the same yield in the double glucosidation reaction. Finally the protecting groups, benzoyls and t-butyl-dimethylsilyl, were deprotected using the same procedure as Zhang et al.

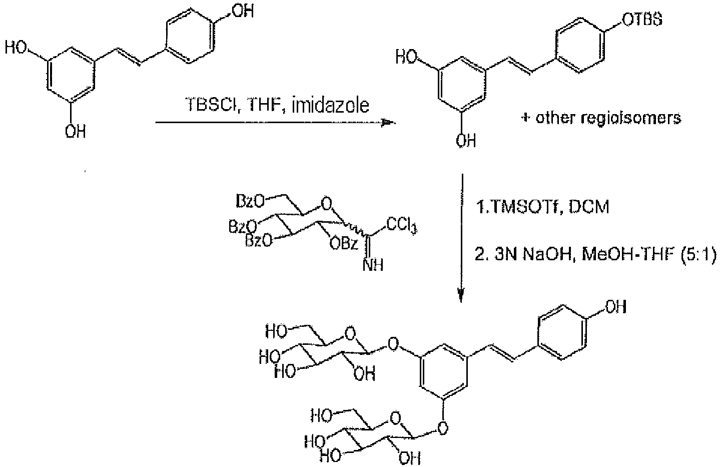

The abbreviations have the following meanings:
TBSCl: t-butyl-dimethylsilyl chloride,
TMSOTf: trimethylsilylester of trifluoromethanesulfonic acid
THF: tetrahydrofuran
DCM: dichloromethane
MeOH: methanol

### Synthesis of the piceid-butyrate compound (trans-resveratrol-3-O-(6'-O-butanoyl)-β-D-glucopyranoside, BUT).

The piceid-butyrate was synthesized through the reaction of piceid (resveratrol-3-*O*-glucoside) with vinyl butyrate in the presence of an immobilized lipase using t-butanol (t-BuOH) as solvent (see diagram). The lipase from *Thermomyces Ianuginosus* immobilized in porous granulated silica marketed by the company Novozymes A/S under the name *Lipozyme TL IM^{®}* was used. The reaction was carried out at 60°C with orbital stirring for 14 hours. The reaction mixture was purified by column chromatography to yield trans-resveratrol-3-*O*-(6'-*O*-butanoyl)-β-D-glucopyranoside (also known as piceid-butyrate or BUT) in very high yield (95-97%).

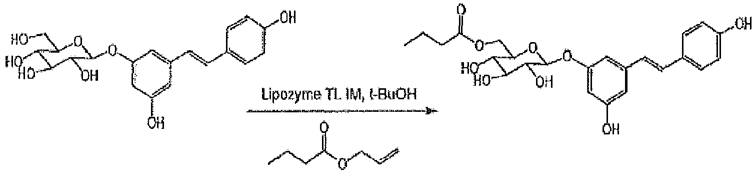

### Characterization

¹H-RMN (MeOD, 300 MHz), δ ppm: 7.38 (d, 2H, J = 8.4 Hz); 7.03 (d, 1H, J = 16.2 Hz); 6.86 (d, 1H, J = 16.2 Hz); 6.79 (d, 2H, J = 8.7 Hz); 6.74 (s, 1H); 6.64 (s, 1H); 6.43 (t, 1H, J = 2.1 Hz); 4.92 (m, 1H), 4.45 (dd, 1H, J = 2.1 and 12 Hz); 4.28-4.21 (m, 1H); 3.73-3.67 (m, 1H); 3.53-3.47 (m, 2H); 3.37 (m, 1H), 2.30 (t, 2H, J = 7.5 Hz); 1.59-1.52 (m, 2H); 0.83 (t, 3H, J = 7.2 Hz); ¹³C-RMN (MeOD, 75 MHz), δ ppm: 174.1; 158.8; 158.2; 157.0; 139.9; 128.8; 128.6; 127.5; 125.4; 115.2; 107.0; 105.5; 102.9; 100.5; 76.5; 73.9; 73.4; 70.5; 63.4; 35.5; 17.9; 12.5; HRESIMS: calculated for C₂₄H₂₈NaO₉ 483.1631, found 483.1648.

### Synthesis of the piceid-octanoate compound (trans-resveratrol-3-O-(6'-O-octanoyl)-β-D-glucopyranoside, OCT).

The synthesis of piceid-octanoate or trans-resveratrol-3-*O*-(6'-*O*-octanoyl)-β-D-glucopyranoside follows the same procedure as used for the preparation of piceid-butyrate, but using vinyl octanoate as the acylating agent.

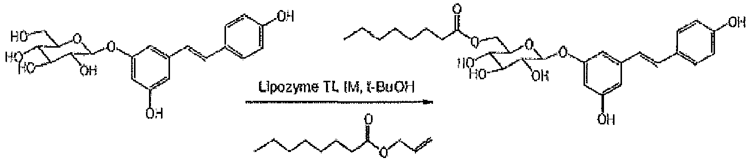

As in the synthesis of the previous compound very high yields were obtained (95-97%).

### Characterization

¹H-RMN (MeOD, 300 MHz), δ ppm: 7.38 (d, 2H, J = 8.7 Hz); 7.03 (d, 1H, J = 16.2 Hz); 6.86 (d, 1H, J = 16.2 Hz); 6.78 (d, 2H, J = 8.7 Hz); 6.74 (s, 1H); 6.64 (s, 1H); 6.43 (t, 1H, J = 2.1 Hz); 4.90 (d, 1H, J = 7.3 Hz), 4.45 (dd, 1H, J = 1.8 and 11.7 Hz); 4.28-4.21 (m, 1H); 3.73-3.68 (m, 1H); 3.51-3.48 (m, 2H); 3.45-3.33 (m, 1H); 2.30 (t, 2H, J = 7.5 Hz); 1.52-1.47 (m, 2H); 1.25-1.16 (m, 8H); 0.86 (t, 3H, J = 7.2 Hz); ¹³C-RMN (MeOD, 75 MHz), δ ppm: 174.1; 158.8; 158.2; 157.1; 139.9; 128.8; 128.5; 127.8; 127.5; 125.4; 115.1; 107.0; 105.2; 100.5; 76.5; 73.9; 73.4; 70.6; 63.4; 33.6; 31.4; 28.9; 28.6; 24.6; 22.2; 13.0; HRESIMS: calculated for C₂₈H₃₆NaO₉ 539.2257, found 539.2269.

### EXAMPLE 2A.- Anti-inflammatory activity of piceid, resveratrol-3,5-O-diglucoside, piceid-butyrate and piceid-octanoate in an animal model of ulcerative colitis. Comparison with resveratrol.

### ANIMAL MODEL AND DISEASE

The animal experiments were carried out in compliance with the principles of the Helsinki Declaration and were authorized by the Animals Department of the University of Murcia. After the experiments the animals were anesthetized with ketamine and xylacin and sacrificed by exsanguination.

A model for intestinal inflammation based on the administration of DSS (sodium dextran sulfate) to C57BL/6 mice was used. At the present time there is no consensus about which overall is the best model for experimental colitis. The DSS model has been widely used because it has clinical, morphological and analytical characteristics which up to a point are similar to those specific to patients with ulcerative colitis or Crohn's disease.

The in vivo inflammation test was carried out using duplicates separated by a number of months. In each test each group of animals (n = 8) was fed a standard diet supplemented with 2.3 mg/day/kg (animal weight) of each of the compounds, depending upon the group. This dose assumes the ingestion of 0.05 mg/day (50 µg/day) per mouse (having a mean weight of 22 grams). This dose extrapolated to a human being is equivalent to 10 mg in an adult weighing 70 kg; using the formula HED (human equivalent dose) = animal dose in mg/kg x (animal weight kg/human weight in kg)^{0.33}.

In each test the mice were fed for 3 weeks during which 1% DSS was included in their drink. The DSS was maintained for 8 days (and the same food as from the start). Then the DSS was withdrawn and the animals were allowed to recover for another 6 days (again with the same food).

Groups included in the investigation: control (standard food), control-DSS, DSS-resveratrol (RES), DSS-piceid (PIC), DSS-resveratrol-diglucoside (DIGLUC), DSS-piceid-butyrate (BUT), DSS-piceid-octanoate (OCT).

The effects of the induced colitis, as previously described in this model, were as follows:
- Change in the mucosal barrier increasing exposure of macrophages to microbiota.
- Release of pro-inflammatory cytokines.
- Increased output of prostaglandins.
- Loss of appetite and weight.
- Diarrhea and blood in feces. Anemia.
- Shortening of the colon and thickening of its walls. Glucoside infiltration, loss of epithelium and destruction of crypts.
- Increase in intestinal and systemic inflammatory status.
- Change in the microbiota with an increase in the number of enterobacteria.

### EVALUATIONS MADE:

- **Monitoring of weight, food and drink ingestion.** The mice in all groups were individually monitored, with weight, quantity of food ingested and water drunk each day being monitored throughout the experimental test.
- **Blood and water in feces.** The water content of feces, as an indication of diarrhea, was evaluated using the difference in weight following evaporation in a stove. Blood was measured using reactive strips based on the guaiacol-peroxidase reaction (HealthCare Diagnosis Inc.). The samples were diluted 1:50 in saline solution and applied to the reactive strips. After 60 sec the color was compared with the template provided by the manufacturer. The reaction was measured using a scale from 0 (negative result) to 4 (most intense color change).
- **Length of colon.** After sacrifice the colon was obtained from each mouse, extended and measured. Samples of the colonic mucosa for various tests mentioned below were obtained by scraping.
- **Myeloperoxidase activity in colonic mucosa.** This was evaluated by spectrophotometry at 460 nm using O-dianisidine reagent and hydrogen peroxide. The change in absorbance at 460 nm was monitored over 10 minutes in a V-630 spectrophotometer (Jasco, Tokyo, Japan). MPO activity was expressed as mU of activity/mg of fresh tissue, comparing the increase in absorbance with a standard straight line obtained using leukocyte myeloperoxidase (Sigma).
- **Prostaglandins in colonic mucosa.** Levels of PGE₂ were measured in colonic mucosa homogenates using an immunoenzyme method using a Cayman Chem. kit (USA).
- **"Antibody array".** The "antibody array" kit from RayBiotech ("RayBio Mouse Inflammation Antibody Array 1") was used to determine cytokines in colonic tissue samples. The "RayBio Mouse inflammation antibody arrays" are a fast and accurate system for determining the expression profile of multiple cytokines (in this case 40) in a very sensitive way. The sensitivity is 100 times greater than that of the ELISA method. The measurement was made in duplicate following the manufacturer's instructions. Membranes were blocked with blocking buffer and then incubated with 300 µg of protein for 2 hours. Finally the membranes were incubated with a cocktail of antibodies conjugated with biotin and then radish peroxidase marked with streptavidin. The signal was detected by chemoluminescence. Images were captured using a CCD camera fitted to a Biorad Chemidoc XRS imaging station. Signal intensities (densitometry) were analyzed using ScanAlyze software. The mean of the intensities for the positive controls was used to standardize the results. The changes in cytokine levels were expressed as mean ± SD in arbitrary density units. The differences for each cytokine were evaluated by ANOVA followed by a post-hoc Tukey test using SPSS software for Windows version 15.0.
- **Colonic architecture.** Samples of the distal colon, fixed in 10% formalin, dehydrated and embedded in paraffin were examined. The samples were cut into 5 µm sections using a Leica microtome, stained with hematoxylin-eosin and observed under an optical microscope. The degree of colitis was evaluated using Araki's system (Araki et al. Clin. Exp. Immunol. 2000, 119, 264-269), paying attention to loss of epithelium, destruction of crypts and infiltration of inflammatory cells.
- **Systemic inflammation markers .** The serum concentration of haptoglobin was quantified by a spectrophotometric method using the "Phase Range Haptoglobin Assay" kit from Tridelta Development (Ireland). Fibrinogen was measured by Clauss' method (Giffen, P.S. et al. Arch. Toxicol. 2003, 77, 392-402).
- **Microbiota counts in feces .** Feces samples were examined on day 0, before the administration of DSS (day 21) and after 8 days administration of DSS (day 29). The samples were homogenized in water with buffered peptone (1:10) using a "stomacher" (IUL Instrument, Barcelona). Lactobacilli and bifidobacteria were cultured on MRS agar and in the case of the bifidobacteria were further supplemented with 0.5 mg/L dicloxacillin, 3 g/L of Lic and 0.5 g/L of L-cisteine hydrochloride. Enterobacteria were cultured on bilis glucose and violet red agar, and E. coli on "Chromocult coliform Agar". Clostridia were cultured in "Reinforced Clostridia." medium. Plates were incubated at 37°C for 24-48 hours in an anaerobic chamber (Don Whitley Scientific Limited, Shipley, U.K.) (CO2:H2:N2 5:15:80). Microorganism counts were expressed as the logarithm of colony forming units per gram (CFU/g).

### RESULTS OBSERVED (P<0.05):

The results reflect the mean for all the mice in each group and the two independent tests.
   - **Conservation of colon length.** The mice in the BUT and OCT groups showed the least shortening of the colon as a consequence of the administration of DSS (Figure 1).
   - **Weight change.** All the animals followed a normal change in weight curve during the first 3 weeks of being fed with food supplemented with different derivatives of resveratrol with an HED of 10 mg. After the administration of DSS (day 21) weight gain slowed down, with weight loss in most of the groups in comparison with the control animals. However, the mice in the BUT and OCT groups maintained the same weight as the control group. The space between the arrows indicates the period during which DSS was administered (Figure 2).
   - **Evaluation of DAI (Disease Activity Index).** Intestinal inflammation brought about by DAI, which correlated with weight loss, the consistency of feces (water in feces) and the presence of blood in the same were evaluated daily (once DSS had been included in the water, day 21 of the overall test). In the graph days 1 to 8 correspond to the administration of DSS and 8 to 14 to the recovery period. The improvement observed in groups BUT and OCT, which is much less than in the remainder (Figure 3) is marked with an arrow.

The activity index was calculated as the mean score for the variables loss of weight, feces consistency and blood in feces.

Weight loss was calculated as the percentage difference between the original weight on day 0 (before starting the administration of DSS) and weight on each day during which DSS was administered and the recovery days. Scores were as follows:
0: <1% weight loss
1: 1-5% weight loss
2: 5-10% weight loss
3: 10-15% weight loss
4: >15% weight loss

Feces consistency was evaluated on the basis of the percentage water in feces.
0 = normal up to 60% water in feces
1 = rather soft 60-70% water in feces
2 = soft 70-75% water in feces
3 = diarrhea > 75% water in feces

The presence of blood in feces was determined using reactive strips based on the guaiacol test (Bayer, Barcelona, Spain).

### 0: negative; 1 = +; 2 = ++; 3 = +++; 4 = ++++

- **Reduced myeloperoxidase activity in colonic mucosa.** Myeloperoxidase (MPO) is an enzyme secreted by activated monocytes and neutrophils. In intestinal inflammation it measures the degree of infiltration of lymphoid tissue into the mucosa. It has pro-inflammatory power and in cases of cardiovascular disease contributes to direct damage to atherosclerotic plaques. It predicts events in patients with coronary disease. The most obvious fall occurs with the administration of OCT and BUT (Figure 4).
- **Effects on systemic inflammation.** Haptoglobin and fibrinogen, which are very useful markers for acute inflammatory events (they belong to the so-called acute phase proteins) are very useful markers. In all cases a clear tendency to improvement was observed, including RES, DIGLUC, BUT and OCT, the most notable differences with respect to the DSS group being those observed for haptoglobin with BUT and OCT (Figure 5) and for fibrinogen with DIGLUC, BUT and OCT (Figure 6).
- **Attenuation of damage in colonic mucosa.** In the control-DSS massive destruction of the colonic crypts and epithelium as well as substantial infiltration of lymphoid tissue was observed. All the derivatives exerted a protective effect on the above changes. The BUT and OCT derivatives were the ones which best preserved colonic architecture during treatment with DSS (Figure 7).
- **"Antibody array" in colonic mucosa.** (Values after ending the administration of DSS; without recovery period). Only results with statistically significant differences are shown (although a clear tendency was also observed in many other cases). The "antibody array" provided us with a sensitive and reproducible measurement of many proteins simultaneously.
   **TNFR1 (Tumor necrosis factor receptor, CD120a).** This receptor for TNFα measures inflammatory response involving the MAP kinases and NFKappaB route. It measures induction of the powerful pro-inflammatory cytokine IL-6. In the case of BUT and OCT the values are similar to those for the control mice (Figure 8).
   **Macrophage inflammatory protein (MIP).** MIP-γ is an inflammatory protein of macrophages and a powerful attractor of neutrophils. It therefore encourages the recruitment of neutrophils to potentiate the inflammatory response. It induces the production of cytokines such as IL-1, IL-6 and TNFα. It is known that reduction in MIP reduces the harmful consequences of inflammation (Figure 9).
   **MIG.** This cytokine produced by monocytes (monokine) is induced by IFN-γ. MIG (also known as CXCL9) is attracted to T cells and critically influences the inflammatory process. All derivatives reduce the level of MIG in a similar way (Figure 10).
   **Interleukin-3 (IL-3).** IL-3 improves the body's defenses as part of the immune system. It has in fact proved to be useful in patients with cancer undergoing chemotherapy because it stimulates the differentiation of multipotent hematopoietic cells. IL-3 is not associated with inflammatory processes (the value was unchanged after treatment with DSS), but it was included because an increase in it was considered to be a positive effect. All derivatives had a statistically significant effect (Figure 11).
   **Interleukin-6 (IL-6).** IL-6 is the key pro-inflammatory cytokine in acute and chronic inflammation processes. It is involved in thrombotic/inflammation processes because it activates coagulation. It is also related to obesity and insulin resistance (Figure 12).
- **Prostaglandin synthesis.** Prostaglandin E2 is involved in inflammation, pyrexia (fever) and hypersensitivity to pain. This is the final product formed from arachidonic acid and involves key enzymes for its synthesis such as cyclooxygenase-2 (COX-2). All the derivatives had a positive effect decreasing the production of prostaglandins and the expression of COX-2 (at the gene and protein levels). Again the BUT and OCT derivatives were the ones showing the best results (Figure 13).
- **General appearance of the mice.** After the administration of 1% DSS for 8 days the animals which had not ingested any resveratrol derivative manifested ataxia, loss of hair and loss of gloss, thinness and rectal bleeding (Figure 14A). The animals which had been previously fed with resveratrol derivatives showed a substantial improvement in comparison with controls (Figure 14B, C, D), the improvement being observed to be in the following order: OCT (D) ≈ BUT (C) » RES ≈ DIGLUC ≈ PIC (B) » control-DSS (A) (Figure 14). Representative results for the groups having the greatest effect (BUT and OCT) are shown with the references for resveratrol (RES) and control-DSS.
- **Evaluation of microbiota in feces before and after the administration of DSS.** An increase in the bifidobacteria count during the 3 weeks prior to the administration of DSS in comparison with the control group was observed in all the groups (Figure 15). After 3 weeks the highest count was observed in the group with the diet supplemented with DIGLUC, although the differences between the groups were very small. However, after administration of DSS only the OCT and BUT groups continued the upward trend in bifidobacteria counts. On the other hand, in the remaining groups, counts fell drastically to the level of the control group, except in the RES group with slightly higher counts (Figure 15). Slight differences aside, the same result was observed in the case of lactobacilli (Figure 16) and clostridia (Figure 17). In the latter case only the more significant groups (control, RES, BUT and OCT) were evaluated.

Examination of these results basically shows that pretreatment with OCT and BUT increases the population of bifidobacteria, lactobacilli and clostridia, and this increase is maintained after the administration of DSS, which has a known effect in changing intestinal microbiota. The beneficial role of bifidobacteria and lactobacilli is known to stimulate defenses against stress or infection situations and contribute to general intestinal homeostasis. In the case of clostridia it is known that they produce short chain fatty acids, which have known anti-inflammatory effects in the intestine and which may also contribute to the overall effect.

Maintenance of the populations of the bacterial groups examined in BUT and OCT reflects the good intestinal homeostasis status presented by these mice, in addition to the beneficial effects previously described.

DSS is also known to facilitate the growth of enterobacteria, including *E*.*coli*, partly due to the destruction of other groups of microorganisms such as those previously mentioned. A count was performed after the administration of DSS for the most important treatments, BUT and OCT, using resveratrol (RES) and the control as comparisons (Figure 18). A clear reduction in enterobacteria and *E*. *coli* was observed in the three groups, especially in the case of BUT and OCT.

### EXAMPLE 2B. Time of arrival and detected concentration of resveratrol in the colon following the administration of piceid (PIC), resveratrol-3,5-O-diglucoside (DIGLUC), piceid-butyrate (BUT) and piceid-octanoate (OCT) in healthy mice. Comparison with resveratrol (RES).

In another different test, using the same strain of mice as in the previous example but without inducing inflammation, the corresponding compounds were administered using a gastric probang. Each mouse received the same quantity of resveratrol in mol/mol quantities from the various compounds. Of course the same quantity was not administered because in a weight/weight comparison molecules of greater mass contain proportionately less resveratrol. If resveratrol alone provides 1 unit of resveratrol, the relationship is 0.58 for PIC, 0.41 for DIGLUC, 0.5 for BUT and 0.44 for OCT. This relationship assumes the administration of 84 mg/kg of resveratrol per live weight of mouse (HED 0.5 g in a 70 kg person), and this quantity of resveratrol was provided by 145 mg/kg of PIC, 205 mg/kg of DIGLUC, 168 mg/kg of BUT and 191 mg/kg of OCT.

The mice were divided into groups (n = 3) which were sacrificed at different times (15 min, 30 min, 1, 2, 4, 8, 12 and 24 hours) after ingestion of the corresponding compounds. Thus 40 groups of mice of 3 mice each were established. The animals were sacrificed as specified in the previous example.

**Extraction and analysis of metabolites in the gastrointestinal tract.** Colon contents were extracted using methanol:water (50:50), centrifuged and the supernatants were analyzed by high resolution chromatography (Agilent 1200 series) with capillary flow, coupled to a mass spectrometer equipped with an ion trap (ESI) (Bruker Daltonics). The chromatographic peaks were identified on the basis of their ultraviolet spectra, intact ions and daughter ions resulting from their fragmentation. The peaks were quantified at 320 nm using the corresponding standards.

### RESULTS OBSERVED

Resveratrol administered as such (RES) began to be detected in the colon one hour after ingestion (Figure 19), showing a maximum around 5 hours. Structural modifications of resveratrol resulted in a greater presence of the latter in the colon, this being a maximum in the case of the compounds BUT and OCT. The structural modifications introduced into BUT and OCT protected the resveratrol against absorption and conjugation, 6 and 5.6 times more resveratrol being detected in the colon than when resveratrol alone (RES) was used (Figure 19). Thus in compound of general formula (I) the use of an acyl group (-OCO-R₃) for R₂, in which R₃ is preferably a (C₂-C₁₀) alkyl group, and more preferably R₃ is a (C₃-C₇) alkyl group, made it possible to vehicle the resveratrol more effectively to increase its concentration in the colon and therefore to exert a greater biological action.

### EXAMPLE 3 - Anti-adhesion and immunomodulating activity of resveratrol, piceid, resveratrol-3,5-O-diglucoside, piceid-butyrate and piceid-octanoate against pathogenic intestinal bacteria

The ability of the compounds tested to inhibit the adhesion of bacteria such as *Salmonella, E.coli* serotype O157 and *Listeria monocytogenes,* which are three of the main bacterial pathogens involved in food poisoning and infections, helps to prevent intestinal invasion by these bacteria and therefore they are likely to be a very useful tool as inhibitors of mechanisms of bacterial infection. As a consequence they could offer an alternative to prophylaxis using antibiotics. The ability of these compounds to modulate the production of pro-inflammatory molecules such as interleukin-8 (IL-8) as a response to infection by enteropathogenic bacteria such as those previously mentioned would make it possible to prevent intestinal inflammation exacerbated in the presence of these bacteria. As a consequence it would reduce the symptoms associated with infections of the digestive tract.

The two main aspects evaluated in infection by pathogenic bacteria, adhesion of the bacteria to the host cell and the inflammation caused in that host cell by invasion of the pathogen, will be considered separately.

### ANTI-ADHESION ACTIVITY

### PROCEDURE

We used two cell models for colon cancer to evaluate the anti-adhesion capacity of compounds derived from resveratrol against pathogenic bacteria in food: Caco-2 (a model frequently used to measure the adhesion of microorganisms) and HT-29 (a model frequently used to measure immune response to infections by enteropathogens). Caco-2 and HT-29 cells were grown in EMEM and DMEM respectively, for which 24 well plates were used at 37°C, 5% CO₂, 95% air until a single layer of confluent epithelium formed (cell concentration per well 2 x 10⁵ and 6 x 10⁵ respectively). Pathogenic bacteria (*E. coli* O157, *Salmonella* and *Listeria monocytogenes* Scott A) were placed in contact with the various compounds derived from resveratrol for 1 hour and subsequently the bacteria together with the different compounds in a concentration of 25 µM/well were inoculated on the cell cultures, incubating the plates for 2 hours at 37°C, 5% CO₂, 95% air. The plates were then washed 3 times with 1 ml of sterile PBS and 1 ml of distilled water with 20% of glycerol was added, and they were frozen at -70°C to lyse the epithelial cells. A count of the pathogenic bacteria adhering to the cells was then made using Petri dishes with nutrient agar (for the count for *Salmonella* and *E. coli* O157) and dishes with cerebral-heart infusion agar (for the L. *monocytogenes* count). Concentrations of the various derivatives tested were detected in the digestive tracts of experimental animals and therefore the test lay within the range of physiologically achievable concentrations.

### Results

In the tests using cell cultures of Caco-2 it was observed that resveratrol and its derivatives reduced the adhesion of pathogenic bacteria to the intestinal epithelium when compared with the adhesion of bacteria inoculated without the addition of those compounds (positive control, C+) (Figure 20). The anti-adhesion efficacy of the various derivatives of resveratrol tested was similar in respect of *E*. *coli* O157. However, in respect of *Salmonella* and *L*. *monocytogenes* greater anti-adhesion efficacy was observed for BUT and OCT in comparison with resveratrol and the other derivatives (Figure 20). The tests using cell cultures of HT-29 provided corroboration that resveratrol and its derivatives reduced the adhesion of pathogenic bacteria to intestinal epithelium when compared with the adhesion of bacteria inoculated without the addition of those compounds (positive control, C+) (Figure 21).

### IMMUNOMODULATING ACTIVITY

### Procedure

The human colon carcinoma cell HT-29 model was used to evaluate the ability of the compounds derived from resveratrol to modulate the production of interleukin-8 as a response to pathogenic food bacteria. HT-29 cells were grown in DMEM, and for this purpose 96 well plates were used at 37°C, 5% CO₂, 95% air, until a single layer of confluent epithelium formed. The pathogenic bacteria (*Listeria monocytogenes* Scott A) were placed in contact with the various compounds derived from resveratrol for 1 hour and then the bacteria together with the compounds were inoculated into cell cultures incubating the plates for 6 hours at 37°C, 5% CO₂, 95% air. Cell viability was observed under the microscope and the supernatants from the wells were recovered. After centrifuging (10,000 rpm/10 min) and filtering the supernatants (0.22), IL-8 was determined using the ELISA method (Human Elisa IL-8 diaclone). The concentrations of the various derivatives tested were detected in the digestive tract of experimental animals and therefore the test fell within the range of physiological achievable concentrations.

### Results

The inoculation of *Listeria monocytogenes* into confluent HT-29 epithelium yielded as a result a great production of pro-inflammatory cytokine IL-8, reaching concentrations of 200-250 pg/ml after 6 hours contact (Figure 22). Using this cell model and this pathogen as a representative example of an infection which furthermore progresses with inflammation, the effect of the various derivatives on the production of IL-8 was investigated. Most of the resveratrol derivatives tested were not effective, significantly reducing the production of the cytokines. However OCT and BUT reduced production significantly (Figure 23). This modulation was more obvious when the dose of OCT and BUT was increased up to concentrations of 50 and 100 µM (Figure 23).

### EXAMPLE 4 - Antioxidant activity in fish oils and fish oil emulsions

The evaluation of the compounds' antioxidant activity was tested in fish oil emulsions, a model which simulates activity in membranes and is particularly suitable for simulating antioxidant activity in fish and meat muscle.

A cod liver oil (*Gadus morhua*) provided by Fluka (New-Ulm, Switzerland) was used to carry out the experiments and emulsions of fish oil in water were prepared using lecithin (40% phosphatidylcholine, Sigma) as the emulsifying agent with an oil content of 10%.

The system was enriched with the antioxidant compounds under test (resveratrol, piceid, resveratrol-3,5-diglucoside, piceid-butyrate and piceid-octanoate) and the degree of inhibition of oxidation during induced oxidation experiments in comparison with controls (samples without antioxidants) was compared.

Oxidation of the samples of emulsions was activated thermally in stoves at 40°C. They were monitored daily, and the progress of oxidation was evaluated using the analysis of conjugated hydroperoxides and fluorescent compounds (method proposed by Nielsen et al., 1985; Brit. J. Nutr. 53, 75-86) in emulsions.

The kinetics of formation of the conjugated hydroperoxides and the fluorescent compounds in the different samples were obtained, and the antioxidant activity of the compounds was estimated on the basis of percentage inhibition of the oxidation products formed.

Percentage inhibition was calculated in the propagation stage of oxidation through a modification of the formula proposed by Frankel (1998; Lipid Oxidation. The Oily Press. Dundee, Scotland): percentage inhibition (%): (C-S/C-C₀) x 100, in which C represents the value of the oxidation index in the control, C₀ is the value of the oxidation index in the control at time zero and S is the oxidation value in the samples with compound.

### Results EMULSIONS:

Resveratrol, piceid and the DIGLUC derivative demonstrated significant and very similar antioxidant activity in emulsions (Figure 24) with oxidation inhibitions of between 45 and 60%. These results indicated that DIGLUC can effectively be used to inhibit oxidative rancidity in foods.

The BUT and OCT derivatives demonstrated significant activity in inhibiting oxidative rancidity in fish oil emulsions, with inhibition values of between 55 and 60%, respectively.

## Claims

1. Use of the compound of general formula (I) to prepare a pharmaceutical or food composition for the treatment and/or prevention of inflammatory processes. 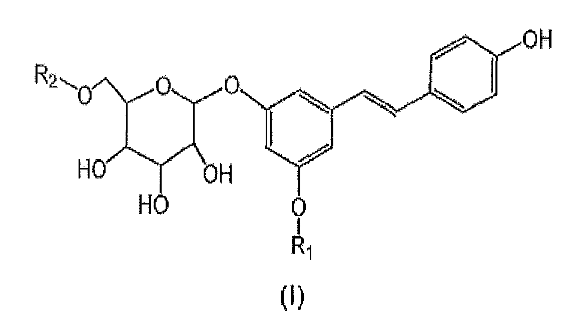 in which:
R₁ is hydrogen or the group of general formula (II): 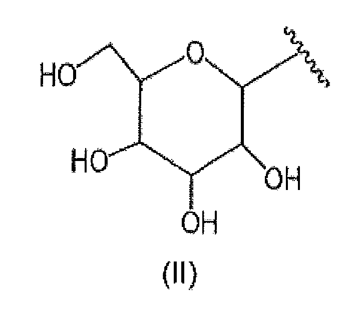
in which: R₂ is hydrogen or together with oxygen forms an acyl group (-OCO-R₃) in which R₃ is a (C₁-C₂₂) alkyl group or a (C₂-C₂₂) alkenyl group, and
when R₂ is hydrogen R₁ represents the group of general formula (II).

2. Use of the compound according to claim 1, in which the pyranose rings forming part of structures (I) or (II) are independently selected from glucose, galactose, mannose or allose.

3. Use of the compound according to either of claims 1 or 2, the union between the pyranose ring present in general formulas (I) or (II) with the phenol ring corresponding to that of alpha or beta stereochemistry.

4. Use of the compound according to any one of claims 1 to 3, in which the pyranose ring is a glucose.

5. Use of the compound according to claim 4, in which the glucose unit is joined to the corresponding phenol ring through beta stereochemistry.

6. Use of the compound according to any one of claims 1 to 5, in which R₁ is hydrogen.

7. Use of the compound according to claims 1 to 6, in which R₂ forms an acyl group and R₃ is a (C₂-C₁₀) alkyl group.

8. Use of the compound according to claims 1 to 7, in which R₃ is a (C₃-C₇) alkyl group.

9. Use of the compound according to any one of claims 1 to 5, in which R₂ is hydrogen, R₁ represents the compound of general formula (II).

10. Use of the compound according to claim 1, selected from the list comprising *trans*-resveratrol-3,5-di-*O*-β-D-glucopyranoside, *trans*-resveratrol-3-*O*-(6'-*O-*butanoyl)-β-D-glucopyranoside and *trans*-resveratrol -3-*O*-(6'-*O*-octanoyl)-β-D-glucopyranoside.

11. Use of the compound according to any one of claims 1 to 10, in which the inflammatory processes derive or originate from inflammatory or systemic diseases, cardiovascular diseases, neurodegenerative diseases or cancer.

12. Use of the compound according to claim 11, in which the systemic inflammatory disease is arthritis.

13. Use of the compound according to any one of claims 1 to 10, in which the inflammatory processes are associated with infectious processes provoked by microorganisms.

14. Use of the compound according to claim 13, in which the microorganisms can be selected from the list comprising viruses, *E. coli* O157, Salmonella *enteritidis* or *Listeria monocytogenes.*

15. Use of the compound according to any one of claims 1 to 10, as food antioxidants.

16. Use of the compound according to any one of claims 1 to 10, in which the inflammatory processes are inflammatory diseases of the digestive tract.

17. Use of the compound according to claim 16, in which the inflammatory diseases are diseases of the intestine.

18. Use of the compound according to either of claims 16 or 17, in which the inflammatory diseases of the digestive tract are selected from the list comprising irritable bowel syndrome, indeterminate colitis, ulcerative colitis and Crohn's disease.

19. Compound of general formula (III) 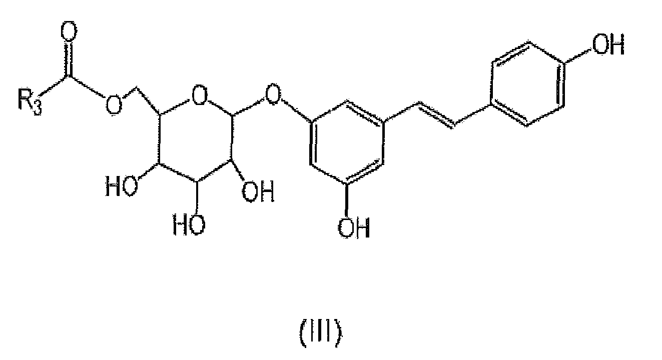 in which: R₃ is described in claim 1.

20. Compound according to claim 19, in which the pyranose ring is a glucose.

21. Compound according to claim 20, in which the glucose is joined to the phenol ring through beta stereochemistry.

22. Compound according to claims 19 to 21, in which R₃ is a (C₂-C₁₀) alkyl group.

23. Compound according to claims 19 to 22, in which R₃ is a (C₃-C₇) alkyl group.

24. Compound according to claim 23, trans-resveratrol-3-*O*-(6'-O-butanoyl)-β-D-glucopyranoside.

25. Compound according to claim 23, trans-resveratrol-3-*O*-(6'-*O*-octanoyl)-β-D-glucopyranoside.

26. Process for obtaining the compounds of formula (III) according to any one of claims 19 to 25, which comprises:
a. mixing compound (IV): 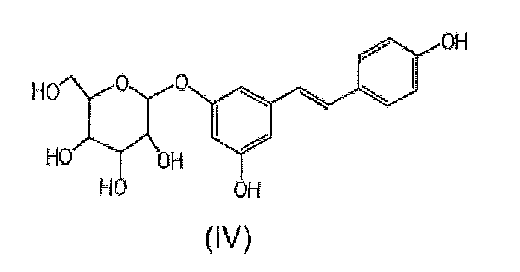 with a compound of formula R₃-COO-R₄ in the presence of a lipase,
in which: R₃ is described in any one of claims 19 to 25 and R₄ is a hydrogen or a group activating the formation of an ester bond.

27. Process according to claim 26, in which the lipase is from *Candida antarctica, Aspergillus niger,* Candida *rugosa, Pseudomonas cepacia, Rhizomucor miehei* or *Thermomyces lanuginosus.*

28. Process according to claim 27, in which the lipase is from *Thermomyces lanuginosus.*

29. Process according to any one of claims 26 to 28, in which the lipase is immobilized.

30. Process according to claim 29, in which the lipase is immobilized in silica.

31. Process according to any one of claims 26 to 30, in which mixing takes place in an organic solvent.

32. Process according to claim 31, in which mixing takes place in acetone, diethylether, diisopropylether, methyl t-butylether, t-butanol, t-amyl alcohol or t-pentyl alcohol or mixtures of one of these with hexane, pentane, cyclohexane, pyridine or toluene.

33. Process according to claim 32, in which mixing of the reagents takes place in t-butanol.

34. Process according to any one of claims 26 to 33, in which the mixture is heated to a temperature between 30°C and 70°C.

35. Process according to claim 26, in which R₄ contains a vinyl group.

36. Use of the compound according to any one of claims 19 to 25 for the preparation of a medication.

37. Use of the compound according to any one of claims 19 to 25 for the preparation of a food composition.

38. Use of the compound according to the preceding claim, in which the food composition can be selected from a foodstuff, a food supplement, a functional foodstuff or nutraceutical.

39. Use of the compound according to any one of claims 19 to 25 as antioxidants.

40. Use of the compound according to the preceding claim to prevent oxidation in foods.

41. Pharmaceutical composition comprising at least one compound of general formula (I) and a pharmaceutically acceptable vehicle.

42. Pharmaceutical composition according to the preceding claim, in which the compound is of general formula (III).

43. Pharmaceutical composition according to either of claims 41 or 42, which also includes another active substance.

44. Food composition comprising at least one compound of general formula (I).

45. Food composition according to the preceding claim, in which the compound is of general formula (III).

46. Use of the compound of formula (I) as described in claim 5 as a vehicle to increase the presence of resveratrol in the intestinal tract.

47. Use of the compound of formula (I) as described in claim 5, to modulate intestinal biota, increasing the intestinal population of bifidobacteria, lactobacilli and decreasing that of the population of pathogens.

48. Use of the compound of general formula (III) according to any one of claims 19 to 25 as a vehicle for increasing the presence of resveratrol in the intestinal tract.

49. Use of the compound of general formula (III) according to any one of claims 19 to 25 to modulate intestinal microbiota, increasing the intestinal population of bifidobacteria, lactobacilli and decreasing the population of pathogens.
